# EUROPEAN PATENT APPLICATION

(11) **EP 4 555 962 A1**
(43) Date of publication of application: **21.05.2025**
(21) Application number: 23849008.0
(22) Date of filing: 26.05.2023
(51) Int. Cl.: A61B 34/30

(54) **SURGICAL OPERATION ARM AND SURGICAL ROBOT**

(30) Priority: 05.08.2022 CN 202210939215
(71) Applicant: Cornerstone Technology (Shenzhen) Limited, Shenzhen Guandong 518066 (CN)
(72) Inventor: WANG, Zerui, Shenzhen, Guangdong 518066 (CN); CAO, Fengyu, Shenzhen, Guangdong 518066 (CN); LIU, Wo Sang, Shenzhen, Guangdong 518066 (CN); TSUI, Yau Ching, Shenzhen, Guangdong 518066 (CN)
(74) Representative: Balder IP Law, S.L.
(86) International application number: PCT/CN2023/096475
(87) International publication number: WO 2024/027298

(57) **Abstract**

Provided are a surgical manipulator and a surgical robot. The surgical manipulator includes a linear drive mechanism (100), a linear transmission mechanism (200), a linear moving mechanism (300), a speed reduction mechanism (400) and a first detection assembly (500). The linear transmission mechanism (200) includes a rotatable transmission member (210) and a mating member (220) threadedly connected to the transmission member (210). The transmission member (210) is connected to the output end of the linear drive mechanism (100), and driven by the linear drive mechanism (100) to rotate. The linear moving mechanism (300) includes a moving block (310) and a linear guide member (320), where the moving block (310) is fixedly connected to the mating member (220), and is movable on the linear guide member (320). The input end of the speed reduction mechanism (400) is fixed to the transmission member (210). The first detection assembly (500) is configured to detect a rotation speed and/or rotation angle and/or rotation position of the output end of the speed reduction mechanism (400).

## Description

### CROSS REFERENCE TO RELATED APPLICATIONS

This claims priority to Chinese Patent Application No. 202210939215.4, entitled "SURGICAL MANIPULATOR AND SURGICAL ROBOT," filed on August 5, 2022, which is incorporated by reference herein in its entirety.

### TECHNICAL FIELD

The present disclosure relates to the technical field of medical instruments, and in particular, to a surgical manipulator and a surgical robot.

### BACKGROUND

A surgical robot can be operated remotely instead of a surgeon to complete a surgery. Since the operation of the surgical robot is stable, a risk of surgical errors, as well as mental and physical stress of the surgeon, can be reduced, and thus, the surgical robot is widely used in minimally invasive surgeries.

Since the surgical robot directly involves life and health safety of the patient, control of the surgical robot to manipulate a surgical instrument to complete a surgery is extremely strict. In some cases, multiple patient-side manipulator arms are prone to mutual interference in the surgeries.

### SUMMARY

Embodiments of the present disclosure provide a surgical manipulator and a surgical robot, avoiding mutual interference of multiple surgical manipulators in the surgeries while ensuring the precision of control of the surgical instrument by the surgical robot.

Embodiments of the present disclosure provide a surgical manipulator. The surgical manipulator includes a linear drive mechanism, a linear transmission mechanism, a linear moving mechanism, a speed reduction mechanism, and a first detection assembly. The linear drive mechanism has a rotating output end. The linear transmission mechanism includes a rotatable transmission member and a mating member threadedly connected to the transmission member. The transmission member is connected to the output end of the linear drive mechanism, and is driven by the linear drive mechanism to rotate about itself. The linear moving mechanism includes a moving block and a linear guide member. The moving block is fixed the mating member of the linear transmission mechanism, and is movable on the linear guide member. The speed reduction mechanism has an input end and an output end. The input end of the speed reduction mechanism is fixed to the transmission member of the linear transmission mechanism, and the transmission member is configured to rotate and drive the input end of the speed reduction mechanism to move. The first detection assembly is disposed at the output end of the speed reduction mechanism and configured to detect at least one of a rotation speed, a rotation angle and a rotation position of the output end of the speed reduction mechanism.

In some embodiments, a transmission ratio of the input end to the output end of the speed reduction mechanism is greater than 1.

In some embodiments, the transmission ratio of the input end to the output end of the speed reduction mechanism satisfies: 10≤i≤50, where i represents the transmission ratio.

In some embodiments, in response to the moving block moving by one full travelling stroke on the linear moving mechanism, the rotation angle of the output end of the speed reduction mechanism is less than 360°.

In some embodiments, the first detection assembly includes an absolute encoder including an absolute encoder fixed portion and an absolute encoder movable portion, where the absolute encoder movable portion is fixed to the output end of the speed reduction mechanism, and the absolute encoder fixed portion is configured to detect at least one of a rotation speed, a rotation angle and a rotation position of the absolute encoder movable portion.

In some embodiments, in response to the moving block moving by one full travelling stroke on the linear moving mechanism, the rotation angle of the output end of the speed reduction mechanism is greater than 360°.

In some embodiments, the first detection assembly includes an incremental encoder including an incremental encoder fixed portion and an incremental encoder movable portion, where the incremental encoder movable portion is fixed to the output end of the speed reduction mechanism, and the incremental encoder fixed portion is configured to detect at least one of a rotation speed, a rotation angle and a rotation position of the incremental encoder movable portion.

In some embodiments, the surgical manipulator further includes a second detection assembly configured to detect at least one of a rotation speed and a rotation angle of the output end of the linear drive mechanism.

In some embodiments, the speed reduction mechanism includes a worm wheel and a worm. The worm is fixed to the transmission member and meshes the worm wheel, the worm wheel is the output end of the speed reduction mechanism, and the first detection assembly is configured to detect at least one of a rotation speed, a rotation angle and a rotation position of the worm wheel.

In some embodiments, the speed reduction mechanism includes at least two gear sets.

In some embodiments, the speed reduction mechanism includes a first stage gear set and a second stage gear set. The first stage gear set includes a first gear and a second gear, the first gear is coaxially fixed to the transmission member of the linear transmission mechanism, the second gear meshes with the first gear, and a diameter of the second gear is greater than a diameter of the first gear. The second stage gear set includes a third gear and a fourth gear, the third gear is coaxially fixed to the second gear, a diameter of the third gear is less than the diameter of the second gear, the fourth gear meshes the third gear, a diameter of the fourth gear is greater than the diameter of the third gear, and the fourth gear is the output end of the speed reduction mechanism.

In some embodiments, the speed reduction mechanism includes at least two planetary gear sets.

In some embodiments, the speed reduction mechanism includes a first planetary gear set and a second planetary gear set. The first planetary gear set includes a first sun gear and a plurality of first planetary gears, the first sun gear is coaxially fixed to the transmission member of the linear transmission mechanism, the plurality of first planetary gears mesh the first sun gear, a diameter of each of the plurality of first planetary gears is greater than a diameter of the first sun gear, and the plurality of first planetary gears are connected to a first planetary carrier. The second planetary gear set includes a second sun gear and a plurality of second planetary gears, the second sun gear is coaxially fixedly to an output end of the first planetary carrier, the plurality of second planetary gears mesh the second sun gear, a diameter of each of the plurality of second planetary gears is greater than a diameter of the second sun gear, the plurality of second planetary gears are connected to a second planetary carrier, and an output end of the second planetary carrier is the output end of the speed reduction mechanism.

In some embodiments, the input end of the speed reduction mechanism is directly fixedly connected to, indirectly fixedly connected to, or integrally provided with the transmission member of the linear transmission mechanism.

Embodiments of the present disclosure further provide a surgical robot. The surgical robot includes the surgical manipulator described above, an instrument driver fixedly connected to the moving block of the surgical manipulator, a surgical instrument connected to the instrument driver, and a surgical connecting arm movably connected to an end of the surgical manipulator away from the instrument driver.

In some embodiments, a plurality of surgical connecting arms are provided, and the plurality of surgical connecting arms are movably connected in sequence, where the end of the surgical manipulator away from the instrument driver is movably connected to the surgical connecting arm located at the end.

In the surgical manipulator in the embodiments of the present disclosure, the transmission member is driven by the linear drive mechanism to rotate so as to drive the mating member to linearly displace in an axial direction of the transmission member, which causes the moving block connected to the transmission member to displace accordingly. In addition, the input end of the speed reduction mechanism is fixed to the transmission member, and the rotation speed and/or rotation angle and/or rotation position of the output end of the speed reduction mechanism is detected by the first detection assembly, so that the linear positions of the moving block on the linear guide member which are in one-to-one correspondence with the rotation positions of the output end of the speed reduction mechanism can be determined, and by taking the rotation angle or position of the output end of the speed reduction mechanism as the reference for the rotation of the transmission member driven by the linear drive mechanism, accurate adjustment of the position of the moving block is achievable. In this process, the detection assembly has no need to be disposed in the linear transmission area of the surgical manipulator, which saves the space for the linearly moving part of the surgical manipulator, thereby reducing the size of the surgical manipulator, thus preventing mutual interference of multiple surgical manipulators in the surgical robot, and at the same time ensuring accurate control of the surgical instrument and improving the safety of surgery performed by the surgical robot.

### BRIEF DESCRIPTION OF THE DRAWINGS

To describe the technical solutions in the embodiments of the present disclosure more clearly, the following briefly introduces the accompanying drawings required for describing the embodiments. Those of ordinary skill in the art may still derive other accompanying drawings from these accompanying drawings without creative efforts. One or more embodiments are exemplarily described with reference to the corresponding figures in the accompanying drawings, and the descriptions are not to be construed as limiting the embodiments. Elements in the accompanying drawings that have same reference numerals are represented as similar elements, and unless otherwise particularly stated, the figures in the accompanying drawings are not drawn to scale.
FIG. 1 is a schematic view illustrating the structure of a surgical manipulator according to some embodiments of the present disclosure.
FIG. 2 is a schematic view illustrating part of the structure of a surgical manipulator according to some embodiments of the present disclosure.
FIG. 3 is a schematic view illustrating the structure of a speed reduction mechanism of a surgical manipulator according to other embodiments of the present disclosure.
FIG. 4 is a partially enlarged view of a speed reduction mechanism of a surgical manipulator according to yet other embodiments of the present disclosure.
FIG. 5 is a partial sectional view of a surgical manipulator according to some embodiments of the present disclosure.
FIG. 6 is a schematic view illustrating the structure of a surgical manipulator according to some embodiments of the present disclosure from another viewing angle.
FIG. 7 is a schematic view illustrating part of the structure of a surgical manipulator according to some embodiments of the present disclosure.

### DETAILED DESCRIPTION OF THE EMBODIMENTS

Features of various aspects and exemplary embodiments of the present disclosure will be described in detail below. To make the objects, technical solutions and advantages of the present disclosure clearer, embodiments of the present disclosure are described in detail below in conjunction with the accompanying drawings. Those of ordinary skill in the art should appreciate that embodiments described herein are merely construed as describing the present disclosure, but not limiting the present disclosure. For those of ordinary skill in the art, the technical solutions claimed in the present disclosure can be implemented without some of these specific details. The following description of embodiments is intended merely to provide a better understanding of the present disclosure by illustrating examples of the present disclosure.

It is to be noted that as used herein, relationship terms such as "first" and "second" are used merely to distinguish one entity or operation from another, and does not necessarily require or imply any such actual relationship or order between these entities or operations. Furthermore, the terms "comprising," "including" or any other variant thereof are intended to encompass a non-exclusive inclusion so that a process, method, article or device that includes a series of elements not only includes the expressly listed elements but may further include other elements that are not expressly listed or are inherent to such process, method, article or device. In the absence of more restrictions, the elements defined by the statement "including ..." do not exclude the presence of additional identical elements in the process, method, article or device that includes the elements.

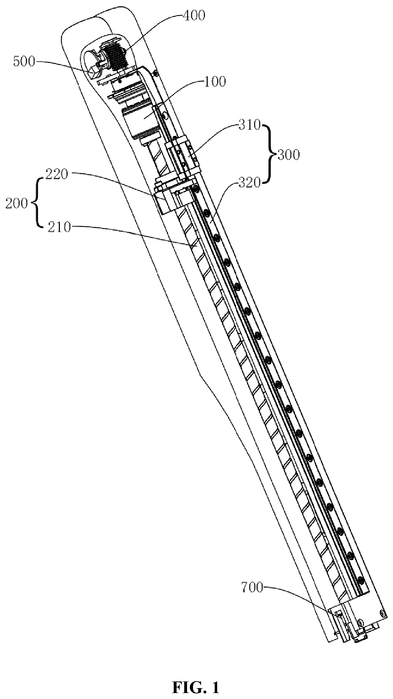

FIG. 1 is a schematic view illustrating the structure of a surgical manipulator according to embodiments of the present disclosure.

As shown in FIG. 1, a first aspect of embodiments of the present disclosure provides a surgical manipulator 10 including a linear drive mechanism 100, a linear transmission mechanism 200, a linear moving mechanism 300, a speed reduction mechanism 400 and a first detection assembly 500. The linear drive mechanism 100 has a rotating output end. The linear transmission mechanism 200 includes a rotatable transmission member 210 and a mating member 220 threadedly connected to the transmission member 210. The transmission member 210 is connected to the output end of the linear drive mechanism 100, and is driven by the linear drive mechanism 100 to rotate about itself. The linear moving mechanism 300 includes a moving block 310 and a linear guide member 320, where the moving block 310 is fixed to the mating member 220 of the linear transmission mechanism 200, and is movable on the linear guide member 320. The speed reduction mechanism 400 has an input end and an output end. The input end of the speed reduction mechanism 400 is fixed to the transmission member 210 of the linear transmission mechanism 200 and is rotatable with rotation of the transmission member 210. The first detection assembly 500 is disposed at the output end of the speed reduction mechanism 400, and is configured to detect a rotation speed and/or rotation angle and/or rotation position of the output end of the speed reduction mechanism 400.

The linear drive mechanism 100 may be a brush motor or a brushless motor, or any other motor such as a servo motor or any device that can drive the rotation of the transmission member 210 of the linear transmission mechanism 200. In other embodiments, the linear drive mechanism 100 may be manually operable, i.e., the way of manually controlling the rotation of the transmission member 210 of the linear transmission mechanism 200 is also within the scope of protection of the present disclosure. Correspondingly, as the linear drive mechanism 100 is a motor, the output end of the linear drive mechanism 100 may be an output shaft or a rotor of the motor.

The transmission member 210 of the linear transmission mechanism 200 may be a lead screw, or a screw rod. Alternatively, the transmission member 210 of the linear transmission mechanism 200 may be a worm or any other device that can be threaded to the transmission member 210.

It is to be understood that the linear moving mechanism 300 including the moving block 310 and linear guide member 320 may be a combination of a sliding block and a sliding rail, or a combination of a moving block 310 and a guide rod. Of course, any other structure that can be used for linear moving guide is within the scope of protection of the present disclosure.

The speed reduction mechanism 400 may be a worm and worm wheel reduction structure, a gear reduction structure, a planetary gear reduction structure, or the like. In other embodiment, the speed reduction mechanism 400 may be a gear and rack engagement structure, a worm and rack engagement structure, or the like. The speed reduction mechanism 400 may be a one-stage speed reduction mechanism, or a multi-stage speed reduction mechanism. It is to be understood that in the speed reduction mechanism 400, based on the detected rotation speed and/or rotation angle and/or rotation position of the output end in combination with a transmission ratio of the input end to the output end, a rotation speed and/or rotation angle and/or rotation position of the corresponding input end can be obtained through calculation or matching.

It is to be understood that in the embodiments of the present disclosure, during movement of the moving block 310, taking the output end of the speed reduction mechanism 400 as a reference, the rotation speed and/or rotation angle and/or rotation position of the output end of the speed reduction mechanism 400 is detected and combined with the transmission ratio of the speed reduction mechanism 400 to calculate a corresponding rotation speed and/or rotation angle and/or rotation position of the input end of the speed reduction mechanism 400. The corresponding rotation speed and/or rotation angle and/or rotation position of the input end of the speed reduction mechanism 400 is equal to a rotation speed and/or rotation angle and/or rotation position of the transmission member 210, based on which a target linear position of the moving block 310 can be calculated. As the rotation positions of the output end of the speed reduction mechanism 400 are in one-to-one correspondence with the linear positions of the moving block 310 on the linear guide member 320, when a movement of the moving block 310 to a target linear position is desired, a rotation of the output end of the speed reduction mechanism 40 to a position corresponding to the target linear position may be employed as a reference for determining if the moving block 310 is moved to the target linear position, thereby facilitating accurate adjustment of the position of the moving block 310.

In the surgical manipulator 10 in the embodiments of the present disclosure, the transmission member 210 is driven by the linear drive mechanism 100 to rotate so as to drive the mating member 220 to linearly displace in an axial direction of the transmission member 210, which causes the moving block 310 connected to the transmission member 210 to displace accordingly. In addition, the input end of the speed reduction mechanism 400 is fixed to the transmission member 210, and the rotation speed and/or rotation angle and/or rotation position of the output end of the speed reduction mechanism 400 is detected by the first detection assembly 500, so that the linear positions of the moving block 310 on the linear guide member 320 which are in one-to-one correspondence with the rotation positions of the output end of the speed reduction mechanism 400 can be determined, and by taking the rotation angle or position of the output end of the speed reduction mechanism 400 as the reference for the rotation of the transmission member 210 driven by the linear drive mechanism 100, accurate adjustment of the position of the moving block 310 is achievable. In this process, the detection assembly has no need to be disposed in the linear transmission area of the surgical manipulator 10, which saves the space for the linearly moving part of the surgical manipulator 10, thereby reducing the size of the surgical manipulator 10, thus preventing mutual interference of multiple surgical manipulators 10 in the surgical robot, and at the same time ensuring accurate control of the surgical instrument 30 and improving the safety of surgery performed by the surgical robot.

In some embodiments, the transmission ratio of the input end to the output end of the speed reduction mechanism 400 is greater than 1. It is to be understood that according to the transmission ratio of the input end to the output end of the speed reduction mechanism 400, through calculation, the angle and/or position of the rotation of the output end of the speed reduction mechanism 400 as the moving block 310 being moved by a predetermined distance on the linear guide member 320 to arrive at the target linear position can be obtained. In the case of the transmission ratio of the input end to the output end of the speed reduction mechanism 400 being greater than 1 (i.e., the rotation speed of the output end of the speed reduction mechanism 400 is slower than the rotation speed of the input end of the speed reduction mechanism 400, and/or the rotation angle of the output end of the speed reduction mechanism 400 is less than the rotation angle of the input end of the speed reduction mechanism 400 within the same time period, and/or the rotation position which the output end of the speed reduction mechanism 400 reaches is closer to a start position than that of the input end of the speed reduction mechanism 400 within the same time period), when the moving block 310 moves on the linear guide member 320 by one full travelling stroke, the angle and/or position of the rotation of the output end of the speed reduction mechanism 400 is smaller compared with the case where the transmission ratio is less than or equal to 1, so that when the position of the moving block 310 is adjusted by taking the output end of the speed reduction mechanism 400 as the reference, the position of the moving block 310 is more accurate.

For example, in a case of the transmission ratio of the speed reduction mechanism 400 being 10 and the movement of the moving block 310 to a preset target linear position being desired, in response to the transmission member 210 being driven by the linear drive mechanism 100 to rotate by, for example, 10 revolutions to make the moving block 310 move to the target linear position, the output end of the speed reduction mechanism 400 rotates by only 1 revolution. Therefore, if the output end of the speed reduction mechanism 400 is taken as the reference, when the first detection assembly 500 detects that the output end of the speed reduction assembly 400 has rotated by 1 revolution, the linear drive mechanism 100 is stopped, and at this time, the moving block 310 can accurately reach the target linear position, thereby realizing accurate adjustment of the position of the moving block 310.

It can be understood that in the case of the transmission ratio of the input end to the output end of the speed reduction mechanism 400 being 10, the output end of the speed reduction mechanism 400 rotates by a smaller angle than the transmission member 210, and the moving member moving on the transmission member 210 has a relatively large travelling distance.

In some embodiments, the transmission ratio of the input end to the output end of the speed reduction mechanism 400 satisfies 10≤i≤50, where i represents the transmission ratio. In this embodiment, since the travelling distance of the moving block 310 in the axial direction of the transmission member 210 is relatively long, in adjustment of the linear position of the moving block 310 by taking the rotation angle and/or rotation position of the output end of the speed reduction mechanism 400 as the reference, the transmission ratio i of the speed reduction mechanism 400 may be set to meet: 10≤i≤50, so that the space occupied by the speed reduction mechanism 400 in the entire surgical manipulator 10 can be reduced while enabling the rotation angles and/or rotation positions of the output end of the speed reduction mechanism 400 to be in one-to-one correspondence with the linear positions of the moving block 310.

In other embodiments, in practical applications, the transmission ratio of the input end to output end of the speed reduction mechanism 400 may be less than 10 or greater than 50, depending on the actual situations.

In some embodiments, in response to the moving block 310 moving by one full travelling stroke on the linear moving mechanism 300, the rotation angle of the output end of the speed reduction mechanism 400 is less than 360°. In this embodiment, in the case where the rotation angle of the output end of the speed reduction mechanism 400 is less than 360° in response to the moving block 310 moving by one full travelling stroke on the linear guide member 320, each linear position of the moving block 310 on the linear guide member 320 corresponds to a respective rotation position of the output end of the speed reduction mechanism 400. That is to say, when a movement of the moving block 310 to a certain target linear position on the linear guide member 320 is desired, it is only necessary to drive the transmission member 210 by the linear drive mechanism 100 to rotate to cause a rotation of the output end of the speed reduction mechanism 400 to a rotation position corresponding to the target linear position. In this way, the position of the moving block 310 can be adjusted without multiple calculations for the position of the moving block 310, thereby simplifying the position adjustment of the moving block 310.

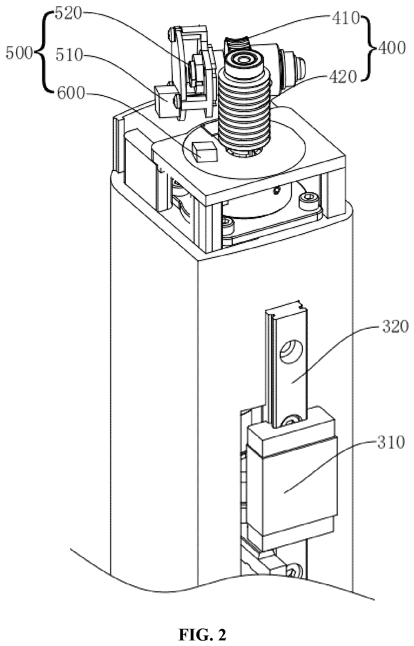

FIG. 2 is a schematic view illustrating part of the structure of a surgical manipulator according to some embodiments of the present disclosure.

As shown in FIG. 2, in some embodiments, the first detection assembly 500 includes an absolute encoder. The absolute encoder includes an absolute encoder fixed portion 510, and an absolute encoder movable portion 520. The absolute encoder movable portion 520 is fixed to the output end of the speed reduction mechanism 400, and the absolute encoder fixed portion 510 is configured to detect a rotation speed and/or rotation angle and/or rotation position of the absolute encoder movable portion 520.

It is to be understood that the absolute encoder has characteristics such as absolute position uniqueness, resistance to interference, and no need for power-off memory. In some embodiments, a code disk of the absolute encoder has a plurality of marks, and each mark corresponds to one respective rotation position of the output end of the speed reduction mechanism 400. Therefore, when the rotation position of the output end of the speed reduction mechanism 400 is detected by the absolute encoder, it is only necessary to make a one-to-one correspondence between the rotation positions of the output end of the speed reduction mechanism 400 and the linear positions of the moving block 310 on the linear guide member 320 through calculation with the transmission ratio during the initial detection. In this case, the marks on the code disk of the absolute encoder are also in a one-to-one correspondence with the linear positions of the moving block 310 on the linear guide member 320. Thus, in the subsequent adjustment of the position of moving block 310, it is only necessary to detect the rotation position of the absolute encoder movable portion located at the output end of the speed reduction mechanism 400 by means of the absolute encoder fixed portion, and when the absolute encoder movable portion is rotated to a position where a certain mark on the code disk is detected by the absolute encoder fixed portion, the linear drive mechanism 100 stops driving so that the moving block 310 is moved to the target linear position corresponding to the certain mark, without multiple calculations for the position of the moving block 310, thus saving the time for the position calculation of the moving block 310 and simplifying the position adjustment of the moving block 310.

Furthermore, the first detection assembly 500 includes the absolute encoder so that after the linear drive mechanism 100 is powered down and then powered up, the exact position of the moving block 310 on the linear guide member 320 can be acquired based on the value directly read from the absolute encoder, which eliminates the need to detect the position of the moving block 310 before adjusting the position of the moving block 310, thereby simplifying the position adjustment of the moving block 310 and improving the efficiency of the adjustment.

In some embodiments, in response to the moving block 310 moving by one full travelling stroke on the linear moving mechanism 300, the rotation angle of the output end of the speed reduction mechanism 400 is greater than 360°. In this embodiment, since the rotation angle of the output end of the speed reduction mechanism 400 is greater than 360° in response to the moving block 310 moving by one full travelling stroke on the linear moving mechanism 300, the rotation positions of the output end of the speed reduction mechanism 400 are not in one-to-one correspondence with the linear positions of the moving block 310 on the linear guide member 320. Therefore, during each adjustment of the position of the moving block 310, it is necessary to detect the rotation speed and/or rotation angle and/or rotation position of the output end of the speed reduction mechanism 400 by the first detection assembly 500, and perform calculation to obtain the linear position of the moving block 310. In this manner, the possibility of the moving block 310 being moved to a linear position other than the target linear position can be reduced, and the chance of the moving block 310 being positioned incorrectly can be reduced.

Referring back to FIG. 2, in some embodiments, the first detection assembly 500 includes an incremental encoder. The incremental encoder includes an incremental encoder fixed portion and an incremental encoder movable portion. The incremental encoder movable portion is fixed to the output end of the speed reduction mechanism 400, and the incremental encoder fixed portion is configured to detect a rotation speed and/or rotation angle and/or rotation position of the incremental encoder movable portion.

It is to be understood that the incremental encoder refers to an encoder that converts an angular displacement or a linear displacement into a periodic electrical signal and converts the electrical signal into pulses for output. Since the incremental encoder can realize infinite accumulation and measurement over multiple revolutions, when the moving block 310 moves by one full travelling stroke on the linear guide member 320, the rotation speed and/or rotation angle and/or rotation position of the output end of the speed reduction mechanism 400 can be detected regardless of the magnitude of the rotation angle of the output end of the speed reduction mechanism 400, thereby achieving the position adjustment of the moving block 310. The incremental encoder does not have a power-off memory function, and thus needs to be zeroed and calibrated after each detection of the position of the speed reduction mechanism 400. In other words, each time the position of the moving block 310 is to be adjusted, it is necessary to detect the rotation speed and/or rotation angle and/or rotation position of the output end of the speed reduction mechanism 400 by the incremental encoder, and perform calculation to obtain the linear position of the moving block 310. In this manner, the possibility of the moving block 310 being moved to a linear position other than the target linear position can be reduced, and the chance of the moving block 310 being positioned incorrectly can be reduced.

In some embodiments, the rotation speed and/or rotation angle and/or rotation position of the incremental encoder movable portion may be detected by the incremental encoder fixed portion, so that the rotation speed and/or rotation angle and/or rotation position of the input end of the speed reduction mechanism 400, i.e., the transmission member 210, can be obtained by calculation in combination with a transmission ratio of the incremental encoder, finally obtaining the target linear position of the moving block 310 by calculation.

It is to be understood that either the absolute encoder fixed portion or the incremental encoder fixed portion may include an encoder sensor, or any other device for detection of a position and/or speed and/or angle, such as an infrared sensor or an ultrasonic sensor; and either the absolute encoder movable portion or the incremental encoder movable portion may include a grating, a magnet, or any other element that can be detected by the corresponding encoder fixed portion, i.e., either the absolute encoder or the incremental encoder may include any one of a magnetic encoder and a grating encoder.

Referring back to FIG. 2, in some embodiments, the surgical manipulator 10 further includes a second detection assembly 600 configured to detect a rotation speed and/or rotation angle of the output end of the linear drive mechanism 100. The rotation speed and/or rotation angle of the output end of the linear drive mechanism 100 can be detected and then combined with the rotation speed and/or rotation angle of the output end of the linear drive mechanism 400 detected by the first detection assembly 500 to calculate an accurate transmission ratio of the speed reduction mechanism 400, so that in adjusting the linear position of the moving block 310, the target linear position of the moving block 310 can be accurately calculated by means of the accurate transmission ratio, ensuring the accuracy of the position adjustment of the moving block 310.

In some embodiments, the second detection assembly 600 may be an encoder, or any other detection assembly that can detect the rotation speed and/or rotation angle. For example, the second detection assembly 600 may be a magnetic encoder or a grating encoder, and the second detection assembly 600 may be an absolute encoder or an incremental encoder. In other embodiment, the second detection assembly 600 may be any other type of encoder.

In some embodiments, taking the second detection assembly 600 being an encoder as an example, the second detection assembly 600 may include an encoder fixed portion (not shown) and an encoder movable portion (not shown). The encoder movable portion is fixedly connected to the transmission member 210, the input end of the speed reduction mechanism 400, or the output end of the linear drive mechanism 100. The encoder fixed portion is configured to detect a rotation speed and/or rotation angle of the encoder movable portion, which can be combined with the rotation speed and/or rotation angle of the output end of the linear drive mechanism 400 detected by the first detection assembly 500 to calculate an accurate transmission ratio of the speed reduction mechanism 400, so that in adjusting the linear position of the moving block 310, the target linear position of the moving block 310 can be accurately calculated by means of the accurate transmission ratio, ensuring the accuracy of the position adjustment of the moving block 310.

Referring back to FIG. 2, in some embodiments, the speed reduction mechanism 400 includes a worm wheel 410 and a worm 420. The worm 420 is fixed to the transmission member 210, and meshes the worm wheel 410. The worm wheel 410 is the output end of the speed reduction mechanism 400, and a rotation speed and/or rotation angle and/or rotation position of the worm wheel 410 is detected by the first detection assembly 500. In some embodiments, the worm 420 is fixed to the transmission member 210 so that during rotation of the transmission member 210 driven by the linear drive mechanism 100, the worm 420 rotates with the rotation of the transmission member 210, and drives the worm wheel 410 to rotate as a result of the meshing of the worm 420 with the worm wheel 410. In the process of adjusting the position of the moving block 310, taking the worm wheel 410 as a reference, the transmission member 210 is driven by the linear drive mechanism 100 to rotate to cause the rotation of the worm wheel 410 to a position corresponding to the target linear position of the moving block 310, thereby achieving accurate control of the position of the moving block 310.

In some embodiments, the speed reduction mechanism 400 includes at least two gear sets. For example, the speed reduction mechanism 400 may include a first stage reduction gear set and a second stage reduction gear set, or include a first stage reduction gear set, a second stage reduction gear set, and a third stage reduction gear set. The specific number of gear sets and the number of stages may be determined according to the actual situation. In this embodiment, the transmission ratio of the input end to the output end of the speed reduction mechanism 400 is further increased by the at least two gear sets in the speed reduction mechanism 400, and thus, in the process of adjusting the position of the moving block 310 by taking the output end of the speed reduction mechanism 400 as the reference, the position of the moving block 310 can be more accurately controlled.

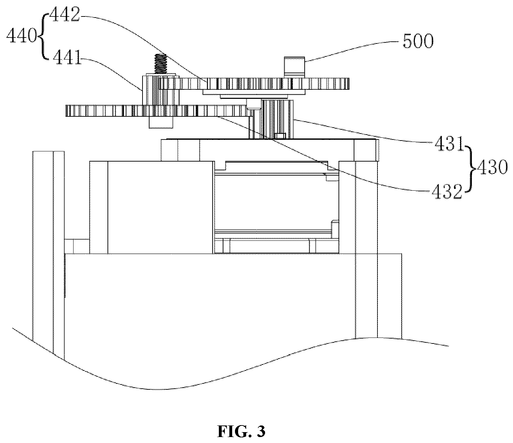

FIG. 3 is a schematic view illustrating the structure of a speed reduction mechanism of a surgical manipulator according to other embodiments of the present disclosure.

As shown in FIG. 3, in some embodiments, the speed reduction mechanism 400 includes a first stage gear set 430 and a second stage gear set 440. The first stage gear set 430 includes a first gear 431 and a second gear 432. The first gear 431 is coaxially fixed to the transmission member 210 of the linear transmission mechanism 200, the second gear 432 meshes the first gear 431, and a diameter of the second gear 432 is greater than a diameter of the first gear 431. The second stage gear set 440 includes a third gear 441 and a fourth gear 442. The third gear 441 is coaxially fixed to the second gear 432, a diameter of the third gear 441 is less than the diameter of the second gear 432, the fourth gear 442 meshes the third gear 441, and a diameter of the fourth gear 442 is greater than the diameter of the third gear 441. The fourth gear 442 is the output end of the speed reduction mechanism 400.

In this embodiment, the first gear 431 meshes the second gear 432, the third gear 441 is coaxially fixed to the second gear 432, and the third gear 441 meshes the fourth gear 442, so that when the transmission member 210 is driven by the linear drive mechanism 100 to rotate, the first gear 431 rotates with the rotation of the transmission member 210 and drives the second gear 432 to rotate, and since the third gear 441 is coaxially fixed to the second gear 432, the third gear 441 is rotated with the rotation of the second gear 432 to drive the fourth gear 442 to rotate, so as to obtain the target linear position of the moving block 310 based on the detected rotation speed and/or rotation angle and/or rotation position of the fourth gear 442 in combination with the calculated transmission ratios between the gears that mesh each other. In this case, since the diameter of the second gear 432 is greater than the diameter of the first gear 431, the diameter of the third gear 441 is less than the diameter of the second gear 432 and the diameter of the fourth gear 442 is greater than the diameter of the third gear 441, the transmission ratio in each of the first stage gear set 430 and the second stage gear set 440 in the speed reduction mechanism 400 is greater than 1, thereby further improving the accuracy of the position adjustment of the moving block 310.

In some embodiments, the speed reduction mechanism 400 includes at least two planetary gear sets. It is to be understood that in this embodiment, the transmission ratio of the input end to the output end of the speed reduction mechanism 400 is further improved by the at least two planetary gear sets in the speed reduction mechanism 400, so that in the process of adjusting the position of the moving block 310 by taking the output end of the speed reduction mechanism 400 as the reference, the position of the moving block 310 can be more accurately controlled.

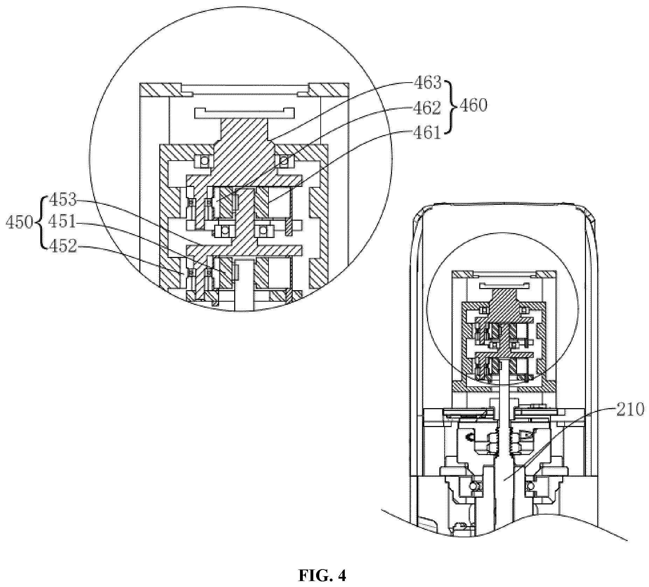

FIG. 4 is a partially enlarged view of a speed reduction mechanism of a surgical manipulator according to yet other embodiments of the present disclosure.

As shown in FIG. 4, in some embodiments, the speed reduction mechanism 400 includes a first planetary gear set 450 and a second planetary gear set 460. The first planetary gear set 450 includes a first sun gear 451 and a plurality of first planetary gears 452. The first sun gear 451 is coaxially fixed to the transmission member 210 of the linear transmission mechanism 200, the first planetary gears 452 mesh the first sun gear 451, a diameter of each of the first planetary gears 452 is greater than a diameter of the first sun gear 451, and the first planetary gears 452 are connected to a first planetary carrier 453. The second planetary gear set 460 includes a second sun gear 461 and a plurality of second planetary gears 462. The second sun gear 461 is coaxially fixedly to an output end of the first planetary carrier 453, the second planetary gears 462 mesh the second sun gear 461, a diameter of each of the second planetary gears 462 is greater than a diameter of the second sun gear 461, and the second planetary gears 462 are connected to a second planetary carrier 463. An output end of the second planetary carrier 463 is the output end of the speed reduction mechanism 400.

In this embodiment, the first sun gear 451 is coaxially fixed to the transmission member 210 and meshes the first planet gears 452, so that when the linear drive mechanism 100 drives the transmission member 210 to rotate, the first sun gear 451 drives the first planet gear 452 to rotate. In addition, since the first planet gears 452 are connected to the first planetary carrier 453 and the second sun gear 461 is coaxially fixed to the output end of the first planetary carrier 453, during the rotation of the first planet gears 452, the first planetary carrier 453 drives the second sun gear 461 to rotate. In addition, since the second sun gear 461 meshes the second planet gears 462 and the second planet gears 462 are connected to the second planetary carrier 463, the second planet gears 462 and the second planetary carrier 463 are driven by the second sun gear 461 to rotate, thereby achieving position adjustment of the moving block 310 based on the detected rotation speed and/or rotation angle and/or rotation position of the fourth gear 442. In addition, since the diameter of each first planet gear 452 is greater than the diameter of the first sun gear 451 and the diameter of each second planet gear 462 is greater than the diameter of the second sun gear 461, the transmission ratio in each of the first stage planet gear set and the second stage planet gear set is greater than 1, thereby further improving the accuracy of the position adjustment of the moving block 310.

In some embodiments, the input end of the speed reduction mechanism 400 is directly fixedly connected to, indirectly fixedly connected to, or integrally provided with the transmission member 210 of the linear transmission mechanism 200.

It is to be understood that the input end of the speed reduction mechanism 400 being indirectly fixedly connected to the transmission member 210 of the linear transmission mechanism 200 may refer to that the input end of the speed reduction mechanism 400 is fixedly connected to the transmission member 210 by a coupling; the input end of the speed reduction mechanism 400 being directly fixedly connected to the transmission member 210 may refer to that the input end of the speed reduction mechanism 400 is directly and rigidly connected to the transmission member 210 in an interference-fit manner without other connection structure for fixation, so that the transmission error between the input end of the speed reduction mechanism 400 and the transmission member 210 can be reduced, improving the transmission accuracy; and the input end of the speed reduction mechanism 400 being integrally provided with the transmission member 210 of the linear transmission mechanism 200 may refer to that the input end of the speed reduction mechanism 400 is at least part of the transmission member 210, thus achieving the synchronized rotation of the input end of the speed reduction mechanism 400 and the transmission member 210 to the greatest extent, reducing the transmission error between the input end of the speed reduction mechanism 400 and the transmission member 210, and improving the transmission accuracy.

In this embodiment, the input end of the speed reduction mechanism 400 is directly fixedly connected to, indirectly fixedly connected to, or integrally provided with the transmission member 210 of the linear transmission mechanism 200 so that the synchronization between the speed reduction mechanism 400 and the transmission member 210 can be achieved, and the structure of the surgical manipulator 10 can be made more compact, thereby reducing the transmission error during the rotation process and improving the transmission precision.

Referring again to FIG. 1, in some embodiments, the surgical manipulator 10 further includes a braking assembly 700. The braking assembly 700 is fixed to the transmission member 210, and configured to stop the transmission member 210 or keep the transmission member 210 in a stopped state. In an embodiment, the braking assembly 700 may be any one of various brakes, such as an electromagnetic brake and a magnetic particle brake. Of course, the braking assembly 700 may also be an other instrument that can stop the first rotating shaft 120 or keep the first rotating shaft 120 in a stopped state, such as a brake or a stop valve.

In this embodiment, the braking assembly 700 is fixedly disposed on the transmission member 210 so that when the moving block 310 reaches the target linear position, the braking assembly 700 stops the transmission member 210 timely, so as to prevent the transmission member 210 from affecting the position accuracy of the moving block 310 due to inertia, and at the same time, the transmission member 210 can be kept in a stopped state by the braking assembly 700 after the moving block 310 has moved to the target linear position, thereby ensuring that the position of the moving block 310 does not change due to an external factor such as gravity or inertia.

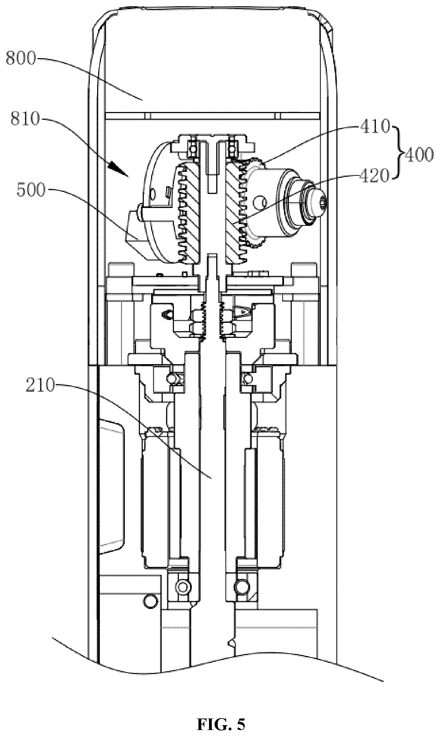

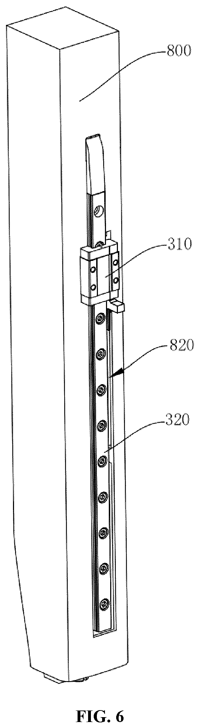

FIG. 5 is a partial sectional view of a surgical manipulator according to some embodiments of the present disclosure. FIG. 6 is a schematic view illustrating the structure of a surgical manipulator according to some embodiments of the present disclosure from another viewing angle.

As shown in FIGS. 5 and 6, in some embodiments, the surgical manipulator 10 further includes an arm body 800 defining an accommodating cavity 810. The arm body 800 is provided with an opening 820 communicating the accommodating cavity 810 with an exterior of the arm body 800. The linear drive mechanism 100, the speed reduction mechanism 400, and the first detection assembly 500 are accommodated in the accommodating cavity 810. The moving block protrudes partially through the opening 820 to the exterior of the arm body 800 to connect to the instrument driver 20.

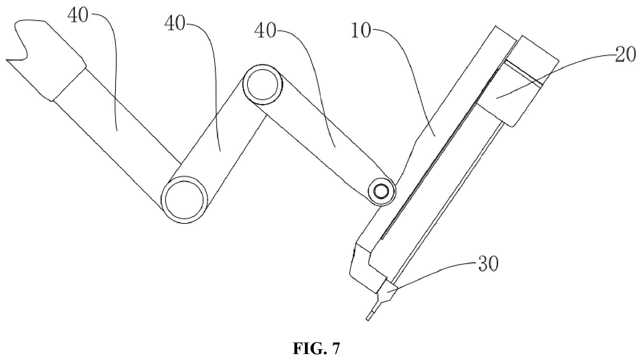

FIG. 7 is a schematic view illustrating part of the structure of a surgical manipulator according to some embodiments of the present disclosure.

As shown in FIG. 7, a second aspect of embodiments of the present disclosure provides a surgical robot. The surgical robot includes the surgical manipulator 10 as described above, an instrument driver 20 fixedly connected to the moving block of the surgical manipulator 10, a surgical instrument 30 connected to the instrument driver 20, and at least one surgical connecting arm 40 movably connected to an end of the surgical manipulator 10 away from the instrument driver 20.

In the surgical robot provided in the embodiments of the present disclosure, the surgical manipulator 10 is connected to the instrument driver 20 so that the accuracy of the displacement of the instrument driver 20 in the axial direction of the transmission member 210 is improved to a certain extent, which ensures the stability and safety of the instrument driver 20 when driving the surgical instrument 30 to perform the surgery. In addition, the end of the surgical manipulator 10 away from the instrument driver 20 is connected to the surgical connecting arm 40 so that the surgical manipulator 10 can have degrees of freedom in other directions through the surgical connecting arm 40, thereby enabling the instrument driver 20 to be more flexible in driving the surgical instrument 30 to perform the surgery.

Referring back to FIG. 7, in order to make the instrument driver more flexible under the control of the surgical manipulator 10, in some embodiments, a plurality of surgical connecting arms 40 are provided, and the plurality of surgical connecting arms 40 are movably connected in sequence, where the end of the surgical manipulator 10 away from the instrument driver 20 is movably connected to the surgical connecting arm 40 located at the end. It is to be understood that the plurality of surgical connecting arms 40 are movably connected in sequence and the end of the surgical manipulator 10 away from the instrument driver 20 is movably connected to the surgical connecting arm 40 located at the end, so that the surgical manipulator 10 can be movable in various directions through the plurality of surgical connecting arms 40, thereby making the instrument driver 20 more flexible under the control of the surgical manipulator 10 and improving the stability and safety of the instrument driver 20 when driving the surgical instrument 30 to perform the surgery.

In the surgical manipulator 10 in the embodiments of the present disclosure, the linear drive mechanism 100 drives the transmission member 210 to rotate and drive the mating member 220 to linearly displace in an axial direction of the transmission member 210, which causes the moving block 310 connected to the transmission member 210 to displace accordingly. In addition, the input end of the speed reduction mechanism 400 is fixed to the transmission member 210, and the rotation speed and/or rotation angle and/or rotation position of the output end of the speed reduction mechanism 400 is detected by the first detection assembly 500. Since the linear positions of the moving block 310 on the linear guide member 320 are in one-to-one correspondence with the rotation positions of the output end of the speed reduction mechanism 400, by taking the positions of the output end of the speed reduction mechanism 400 as the reference for the rotation of the transmission member 210 driven by the linear drive mechanism 100 in the process of adjusting the position of the moving block 310, accurate adjustment of the position of the moving block 310 is achieved, thereby ensuring the accurate control of the surgical instrument 30 and improving the safety of surgery performed by the surgical robot.

The above are merely the embodiments of the present disclosure. It is clearly understood by those skilled in the art that for the convenience and brevity of the description, the specific working processes of the above-described systems, modules, and units can be referred to the corresponding processes in the foregoing method embodiments, which are not be repeated herein, and it is to be understood that the scope of protection of the present disclosure is not limited thereto. Any person skilled in the art can easily conceive of various equivalent modifications or substitutions within the technical scope of the present disclosure, which shall fall into the scope of protection of the present disclosure.

## Claims

1. A surgical manipulator, comprising:
a linear drive mechanism, having a rotating output end;
a linear transmission mechanism, including a rotatable transmission member and a mating member threadedly connected to the transmission member, wherein the transmission member is rotatable about itself, connected to the output end of the linear drive mechanism, and driven by the linear drive mechanism to rotate;
a linear moving mechanism, including a moving block and a linear guide member, wherein the moving block is fixed to the mating member of the linear transmission mechanism, and is movable on the linear guide member;
a speed reduction mechanism, having an input end and an output end, wherein the input end of the speed reduction mechanism is fixed to the transmission member of the linear transmission mechanism, and the transmission member is configured to rotate and drive the input end of the speed reduction mechanism to move; and
a first detection assembly, disposed at the output end of the speed reduction mechanism and configured to detect at least one of a rotation speed, a rotation angle and a rotation position of the output end of the speed reduction mechanism.

2. The surgical manipulator according to claim 1, wherein a transmission ratio of the input end to the output end of the speed reduction mechanism is greater than 1.

3. The surgical manipulator according to claim 2, wherein the transmission ratio of the input end to the output end of the speed reduction mechanism satisfies: 10≤i≤50, wherein i represents the transmission ratio.

4. The surgical manipulator according to any one of claims 1 to 3, wherein in response to the moving block moving by one full travelling stroke on the linear moving mechanism, the rotation angle of the output end of the speed reduction mechanism is less than 360°.

5. The surgical manipulator according to claim 4, wherein the first detection assembly includes an absolute encoder including an absolute encoder fixed portion and an absolute encoder movable portion, wherein the absolute encoder movable portion is fixed to the output end of the speed reduction mechanism, and the absolute encoder fixed portion is configured to detect at least one of a rotation speed, a rotation angle and a rotation position of the absolute encoder movable portion.

6. The surgical manipulator according to any one of claims 1 to 3, wherein in response to the moving block moving by one full travelling stroke on the linear moving mechanism, the rotation angle of the output end of the speed reduction mechanism is greater than 360°.

7. The surgical manipulator according to claim 6, wherein the first detection assembly includes an incremental encoder including an incremental encoder fixed portion and an incremental encoder movable portion, wherein the incremental encoder movable portion is fixed to the output end of the speed reduction mechanism, and the incremental encoder fixed portion is configured to detect at least one of a rotation speed, a rotation angle and a rotation position of the incremental encoder movable portion.

8. The surgical manipulator according to claim 7, further comprising:
a second detection assembly, configured to detect at least one of a rotation speed and a rotation angle of the output end of the linear drive mechanism.

9. The surgical manipulator according to any one of claims 1 to 8, wherein the speed reduction mechanism includes a worm wheel and a worm, wherein the worm is fixed to the transmission member and meshes the worm wheel, the worm wheel is the output end of the speed reduction mechanism, and the first detection assembly is configured to detect at least one of a rotation speed, a rotation angle and a rotation position of the worm wheel.

10. The surgical manipulator according to any one of claims 1 to 8, wherein the speed reduction mechanism includes at least two gear sets.

11. The surgical manipulator according to claim 10, wherein the speed reduction mechanism includes a first stage gear set and a second stage gear set;
wherein the first stage gear set includes a first gear and a second gear, the first gear is coaxially fixed to the transmission member of the linear transmission mechanism, the second gear meshes the first gear, and a diameter of the second gear is greater than a diameter of the first gear; and
wherein the second stage gear set includes a third gear and a fourth gear, the third gear is coaxially fixed to the second gear, a diameter of the third gear is less than the diameter of the second gear, the fourth gear meshes the third gear, a diameter of the fourth gear is greater than the diameter of the third gear, and the fourth gear is the output end of the speed reduction mechanism.

12. The surgical manipulator according to any one of claims 1 to 8, wherein the speed reduction mechanism includes at least two planetary gear sets.

13. The surgical manipulator according to claim 12, wherein the speed reduction mechanism includes a first planetary gear set and a second planetary gear set;
wherein the first planetary gear set includes a first sun gear and a plurality of first planetary gears, the first sun gear is coaxially fixed to the transmission member of the linear transmission mechanism, the plurality of first planetary gears mesh the first sun gear, a diameter of each of the plurality of first planetary gears is greater than a diameter of the first sun gear, and the plurality of first planetary gears are connected to a first planetary carrier; and
wherein the second planetary gear set includes a second sun gear and a plurality of second planetary gears, the second sun gear is coaxially fixedly to an output end of the first planetary carrier, the plurality of second planetary gears mesh the second sun gear, a diameter of each of the plurality of second planetary gears is greater than a diameter of the second sun gear, the plurality of second planetary gears are connected to a second planetary carrier, and an output end of the second planetary carrier is the output end of the speed reduction mechanism.

14. The surgical manipulator according to any one of claims 1 to 8, wherein the input end of the speed reduction mechanism is directly fixedly connected to, indirectly fixedly connected to, or integrally provided with the transmission member of the linear transmission mechanism.

15. A surgical robot, comprising:
the surgical manipulator according to any one of claims 1 to 14; and
an instrument driver, fixedly connected to the moving block of the surgical manipulator;
a surgical instrument, connected to the instrument driver; and
a surgical connecting arm, movably connected to an end of the surgical manipulator away from the instrument driver.
